# EUROPEAN PATENT SPECIFICATION

(11) **EP 1 897 487 B1**
(45) Date of publication and mention of the grant of the patent: **11.11.2009**
(21) Application number: 07253500.8
(22) Date of filing: 04.09.2007
(51) Int. Cl.: A61B 5/00

(54) **Event-driven method for tutoring a user in the determination of an analyte in a bodily fluid sample**
Ereignisgesteuertes Verfahren zur Unterweisung eines Benutzers in der Bestimmung eines Analyts in einer Körperflüssigkeit
Procédé d'événement pour former un utilisateur à déterminer une analyse dans un échantillon de fluide corporel

(30) Priority: 05.09.2006 US 842584 P; 30.10.2006 US 554506
(43) Date of publication of application: 12.03.2008
(73) Proprietor: Lifescan Scotland Ltd, Inverness, IV2 3ED, Scotland (GB)
(72) Inventor: Miyata, Shinichi, San Jose, California 95314 (US); Arndt, Carrie, Pleasanton, California 94566 (US); Sutton, Thomas Rangi, 20125 Milan (IT); Anderson, Gretchen, San Francisco, California 94117 (US)
(74) Representative: Tunstall, Christopher Stephen

(56) References cited:
- EP-A- 0 367 752
- EP-A- 1 279 365
- EP-A- 1 611 836
- WO-A-03/082091
- US-A1- 2004 138 588

## Description

### BACKGROUND OF THE INVENTION

### 1. Field of the Invention

The present invention relates, in general, to medical devices and, in particular, to kits, devices and methods for determining an analyte in a bodily fluid sample.

### 2. Description of the Related Art

The determination (e.g., detection and/or concentration measurement) of an analyte in a bodily fluid sample is of particular interest in the medical field. For example, it can be desirable to determine glucose, cholesterol, acetaminophen and/or HbA1c concentrations in a sample of a bodily fluid such as urine, blood or interstitial fluid. Such determinations can be achieved using kits that employ analytical test strips based on, for example, photometric or electrochemical techniques, and an associated meter (also referred to as an analytical meter). For example, the OneTouch^{®} Ultra^{®} whole blood testing kit, available from LifeScan, Inc., Milpitas, USA, employs an electrochemical-based analytical test strip for the determination of blood glucose concentration in a whole blood sample. Such kits can also, if desired, include a lancing device.

The proper operation of each component of such kits (for example, the combined operation of the lancing device, test strip and meter) can be relatively complex. Therefore, users of such kits are typically provided with at least one written operating manual for the kit. Depending on the complexity of the kit, a user may need to devote significant time and concentration before they understand and have memorized the manual's information and are able to successfully operate each of the kit's components.

Publication US 2004/138588 discloses an analyte testing water with instructions displayed on an LCD.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, and of which:
FIG. 1 is a simplified block diagram of a kit for determining an analyte in a bodily fluid sample according to an exemplary embodiment of the present invention;
FIG. 2 is a simplified front view of an analytical meter and analytical test strip as can be included in kits according to embodiments of the present invention;
FIG. 3 is a simplified side view of the analytical meter of FIG. 2;
FIG. 4 is a simplified flow chart illustrating an exemplary configuration for an event-driven chapter-based display of tutorial images as can be employed in various embodiments of the present invention;
FIGs. 5A through 5G are simplified depictions of portions of tutorial images of a chapter for tutoring a user in the preparation of a lancing device and lancet of a tutorial as can be employed in embodiments of the present invention;
FIGs. 6A through 6F are simplified depictions of portions of tutorial images of a chapter for tutoring a user in test strip insertion of a tutorial as can be employed in embodiments of the present invention;
FIGs. 7A and 7B are simplified depictions of portions of tutorial images of a chapter of for tutoring a user in the lancing of a finger of a tutorial as can be employed in embodiments of the present invention;
FIGs. 8A through 8C are simplified depictions of portions of tutorial images of a chapter for tutoring a user in dosing of a bodily fluid sample (i.e., a whole blood sample) of a tutorial as can be employed in embodiments of the present invention;
FIG. 9A and 9B are simplified depictions of portions of tutorial images of a chapter for tutoring a user in simulated testing of a tutorial as can be employed in embodiments of the present invention;
FIGs. 10A through 10E are simplified depictions of portions of tutorial images of a chapter for tutoring a user in the discarding of a used lancet of a tutorial as can be employed in embodiments of the present invention;
FIGs. 11A through 11C are simplified depictions of portions of tutorial images of a chapter for tutoring a user in the discarding of a used test strip of a tutorial as can be employed in embodiments of the present invention; and
FIG. 12 is a flow diagram depicting stages in a process for tutoring a user in use of a kit for determining an analyte in a bodily fluid sample according to an exemplary embodiment of the present invention.

### DETAILED DESCRIPTION OF EXEMPLARY EMBODIMENTS OF THE INVENTION

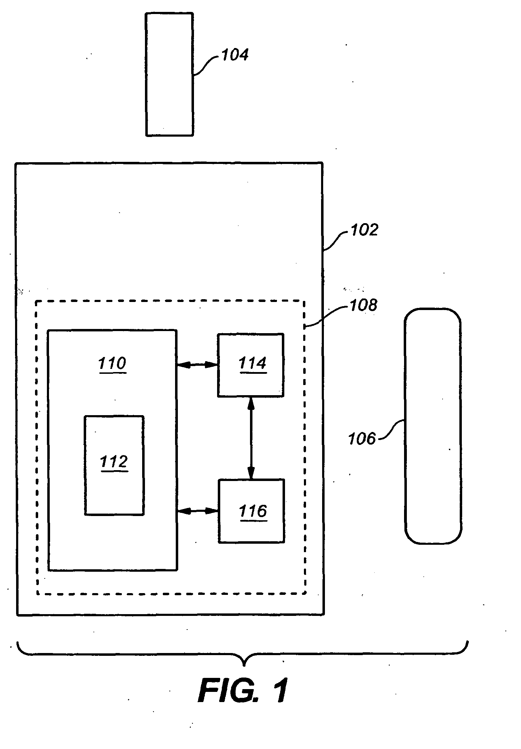

FIG. 1 is a simplified block diagram of a kit 100 for determining an analyte (such as glucose) in a bodily fluid sample (e.g., a whole blood sample) according to an exemplary embodiment of the present invention. Kit 100 includes an analytical meter 102, an analytical test strip 104 and a lancing device 106.

Analytical test strip 104 is configured for the application of a bodily fluid sample thereon and for insertion into analytical meter 102 for subsequent determination of an analyte in the bodily fluid sample. Analytical meter 102 (also referred to simply as a "meter") has a display-based tutorial module 108 (encompassed within the dashed line of FIG. 1) that includes a user interface 110 (with a visual display 112), a memory unit 114 and a microprocessor unit 116.

Memory unit 114 is configured for storing a tutorial, with the stored tutorial having a plurality of chapters. In addition, each of the plurality of chapters contains at least one tutorial image depicting use of the kit. The tutorial images can be stored and arranged into chapters using any suitable techniques known to those of skill in the art including hardware digital image storage and arrangement techniques and/or software-based storage and arrangement techniques. Moreover, memory unit 114 can be any suitable memory unit known to those of skill in the art including, for example, a solid state nonvolatile memory (NVM) units or an optical disk-based memory unit.

Microprocessor unit 116 is configured for controlling and coordinating at least the user interface and the memory unit. Moreover, user interface 110, microprocessor unit 116 and memory unit 114 are operatively linked and configured (as depicted by the doubleheaded arrows of FIG. 1) for event-driven chapter-based display of the tutorial images to a user on visual display 112.

The displayed tutorial images can take any suitable form including, for example, images that are illustrative, pictorial, diagrammatic and/or simplified in nature and are not, therefore, necessarily accurate with respect to all mechanical or visual details and/or in scale. However, such tutorial images are sufficiently accurate and detailed for the intended purpose, namely for the purpose of tutoring a user in the use and operation of a kit or meter for determining an analyte in a bodily fluid sample.

Moreover, such tutorial images can be either static or animated (e.g., an animated sequence of tutorial images) and can, if desired, include image-related text. Tutorial images are images that, for example, exemplify how to properly perform a particular operation in the use of a kit for determining an analyte in a bodily fluid sample or an analytical meter for determining an analyte in a bodily fluid sample. The display of such tutorial images can also beneficially serve to prompt a user, as a reminder, that a particular operational action by the user should be contemplated and/or performed.

Once apprised of the present disclosure, one skilled in the art will recognize that any suitable means can be used for configuring the user interface, microprocessor unit and memory unit to function as described above including the suitable use of electronic circuits, sensors, software and mechanical apparatus as is conventionally known in the art.

Analytical meter 102 can employ any suitable analytical technique or techniques to determine the analyte in the bodily fluid sample including, for example, techniques employed in commercially available meters. Such techniques include, but are not limited to, photometric and electrochemical-based techniques. Once apprised of the present disclosure, one skilled in the art will recognize various manners by which conventional meters could be adapted to implement an embodiment of the present invention. For example, a microprocessor unit, memory unit and a user interface as described herein could be suitably integrated with an otherwise conventional meter to implement an embodiment of the present invention.

User interface 110 of display-based tutorial module 108 can be any suitable user interface and can include, in addition to visual display 112, user operable buttons (not depicted in FIG. 1). Visual display 112 can be, for example, any suitable display screen known to those of skill in the art including a liquid crystal display (LCD) screen. Suitable display screens include, without limitation, display screens that are configured for displaying tutorial images according to the present invention, including static graphics-based images (both with and/or without associated text) and animated graphics-based images (both with and/or without associated text).

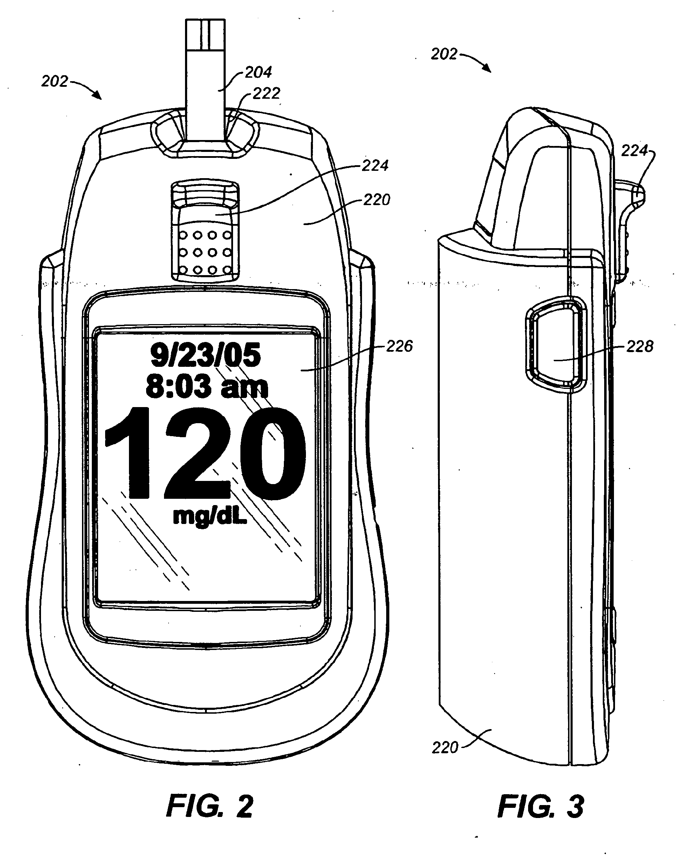

FIG. 2 is a simplified front view of an analytical meter 202 and analytical test strip 204 (shown inserted in analytical meter 202) as can be included in kits according to embodiments of the present invention. FIG. 3 is a simplified side view of analytical meter 202.

Referring to FIGs. 2 and 3, analytical meter 202 includes a housing 220, and a strip port connector 222 for receiving analytical test strip 204. Analytical meter 202 also includes an analytical test strip ejector button 224 and a display-based tutorial module (not entirely shown) as described above with respect to FIG. 1. The display-based tutorial module of analytical meter 202 includes a user interface with a visual display 226 and a user operable tutorial button 228. Other elements of the display-based tutorial module such as the memory unit and microprocessor unit are not visible in the perspective of FIGs. 2 and 3. One skilled in the art will readily comprehend that analytical meter 202 also includes suitable circuitry and sensors for determining an analyte in a bodily fluid sample that has been placed on (also referred to as "dosed") analytical test strip 204.

Although visual display 226 is considered a component of the display-based tutorial module of analytical meter 202, visual display 226 can also be used to perform other functions related to the operation of analytical meter 202. For example, visual display 226 can be used to display a date, time and glucose concentration value as depicted in FIG. 2.

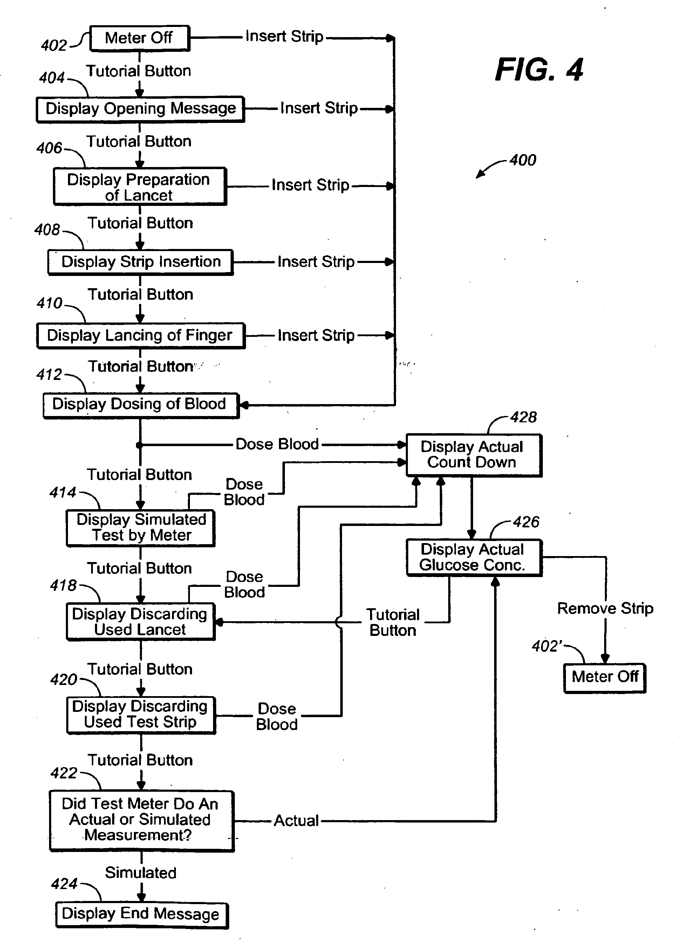

User operable tutorial button 228 is configured such that depression thereof is considered an event by the microprocessor unit of the display-based tutorial module for purposes of displaying tutorial images in an event-driven chapter based manner. As will be clear from the description of FIG. 4 below, an event is rendered unique by its context, i.e., by the other events that have preceded it.

Such unique events and the manner in which they are interpreted by, for example, the microprocessor unit in an event-driven chapter-based display of tutorial images are described in more detail with respect to FIGs. 4 through 11C. Optionally, user operable tutorial button 228 can also be configured such that depression thereof increments the displayed tutorial image within a given chapter of a tutorial. It should also be noted that additional user operable buttons of the user interface can be included in embodiments of the present invention and configured to return to a previously displayed tutorial image within a given chapter of a tutorial.

FIG. 4 is a simplified flow chart illustrating an exemplary configuration 400 for an event-driven chapter-based display of tutorial images as can be employed in various embodiments of the present invention. Configuration 400 illustrates the event-driven chapter-based display of tutorial images for a tutorial that has seven chapters, with each of the chapters containing at least one tutorial image depicting use of a kit for the determination of an analyte (i.e., glucose) in an bodily fluid sample (i.e., a whole blood sample). One skilled in the art will recognize that such chapters are organizational constructs that can be defined within a memory unit, for example, via software and/or via storage of tutorial images to locations within a memory unit that are dedicated to a given chapter.

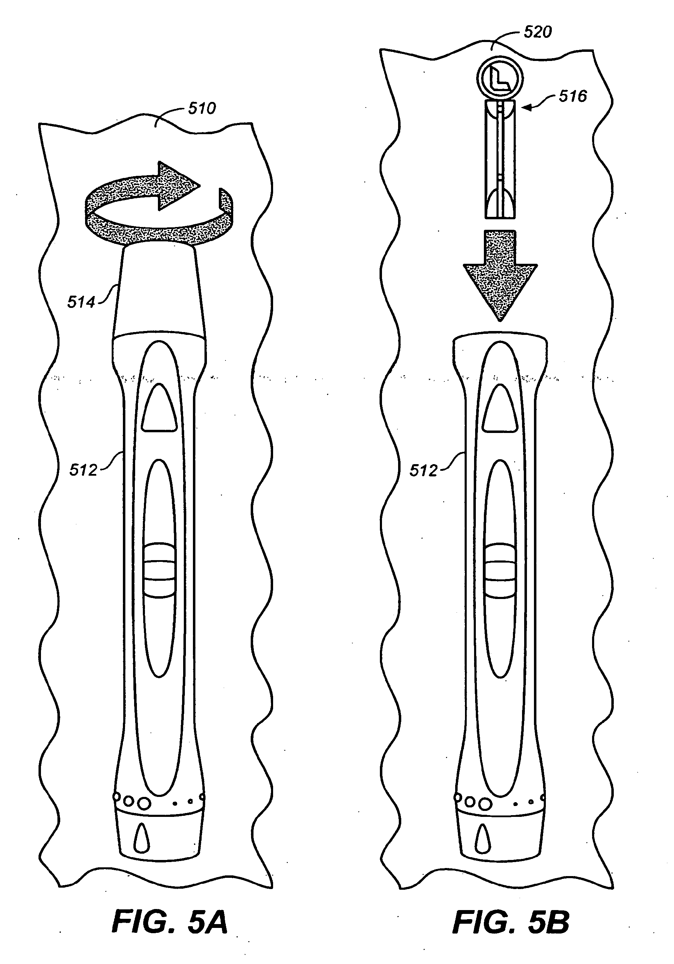

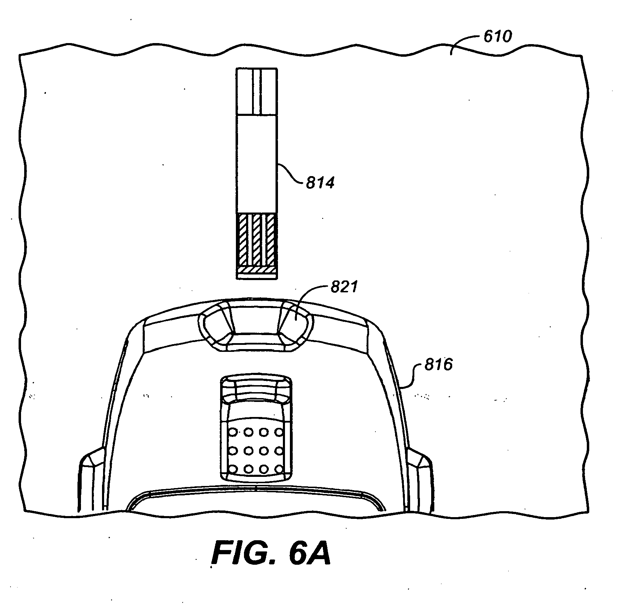

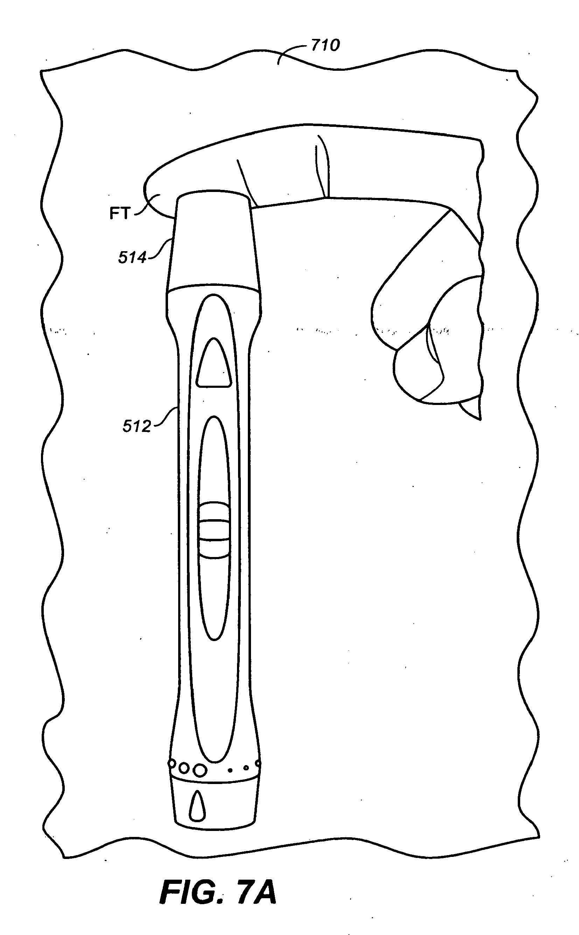

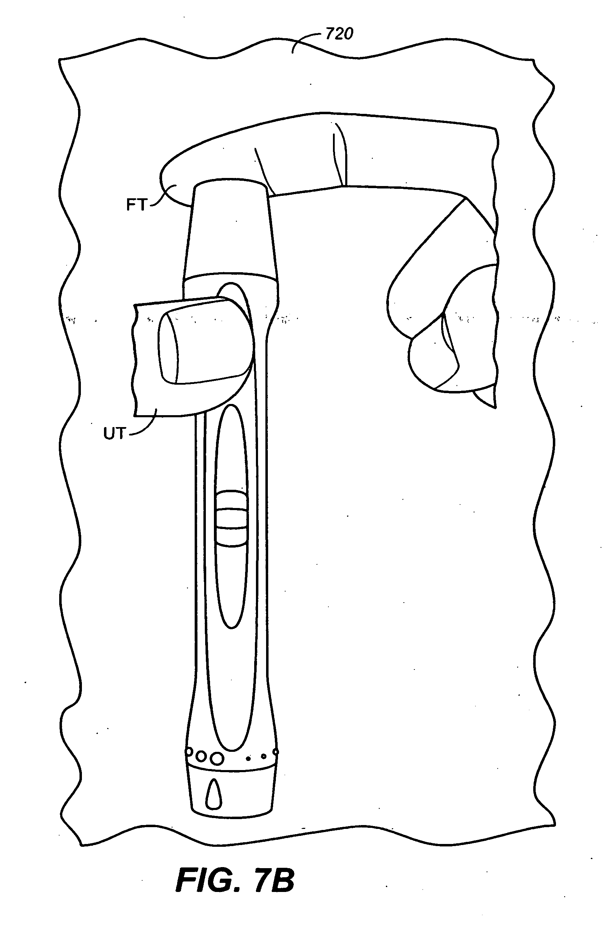

FIGs. 5A through 5G are simplified depictions of portions of seven tutorial images of a chapter for tutoring a user in the preparation of a lancing device and lancet that is referenced in configuration 400 at step 406. FIGs. 6A through 6F are simplified depictions of portions of tutorial images of another chapter for tutoring a user in test strip insertion into a meter that is referenced in configuration 400 at step 408. FIGs. 7A and 7B are simplified depictions of portions of tutorial images of yet another chapter of for tutoring a user in the lancing of a finger that is referenced in configuration 400 at step 410.

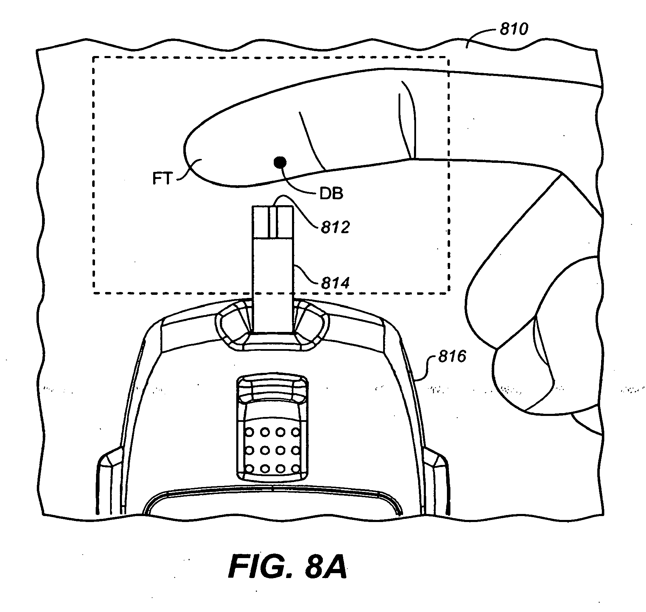

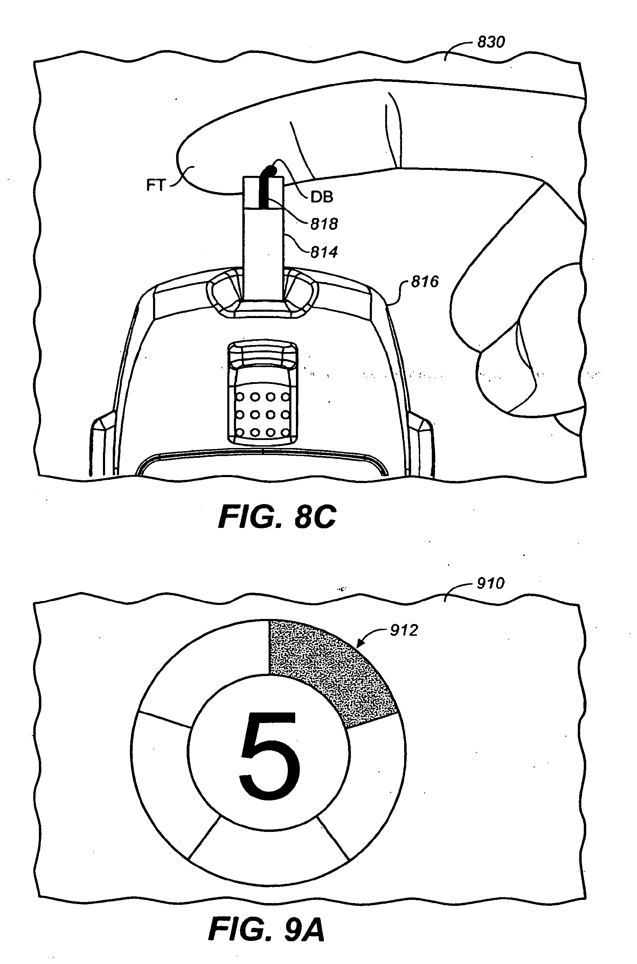

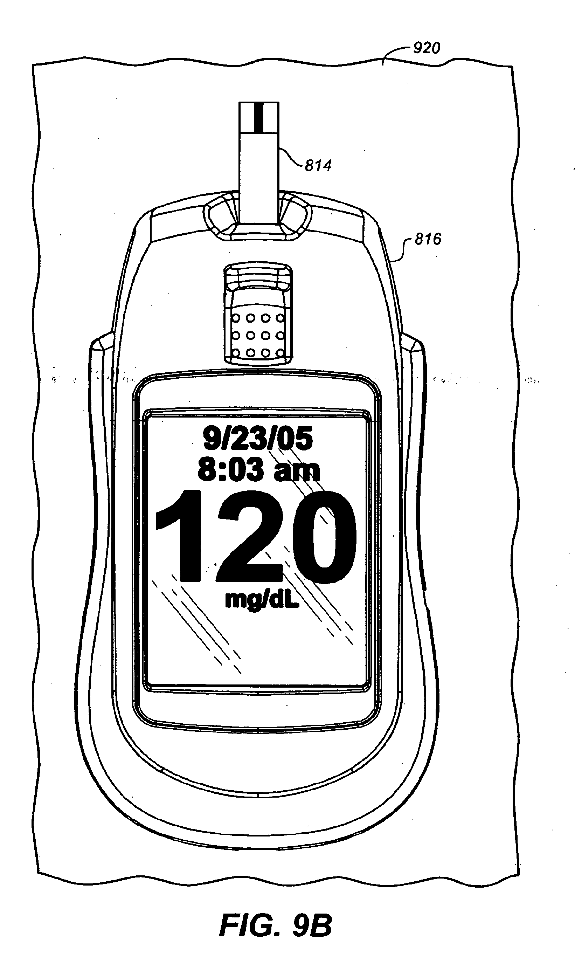

FIGs. 8A through 8C are simplified depictions of portions of tutorial images of a further chapter for tutoring a user in dosing of a bodily fluid sample (i.e., a whole blood sample) that is referenced in configuration 400 at step 412. FIG. 9A and 9B are simplified depictions of portions of tutorial images of an additional chapter for tutoring a user in simulated testing that is referenced in configuration 400 at step 414.

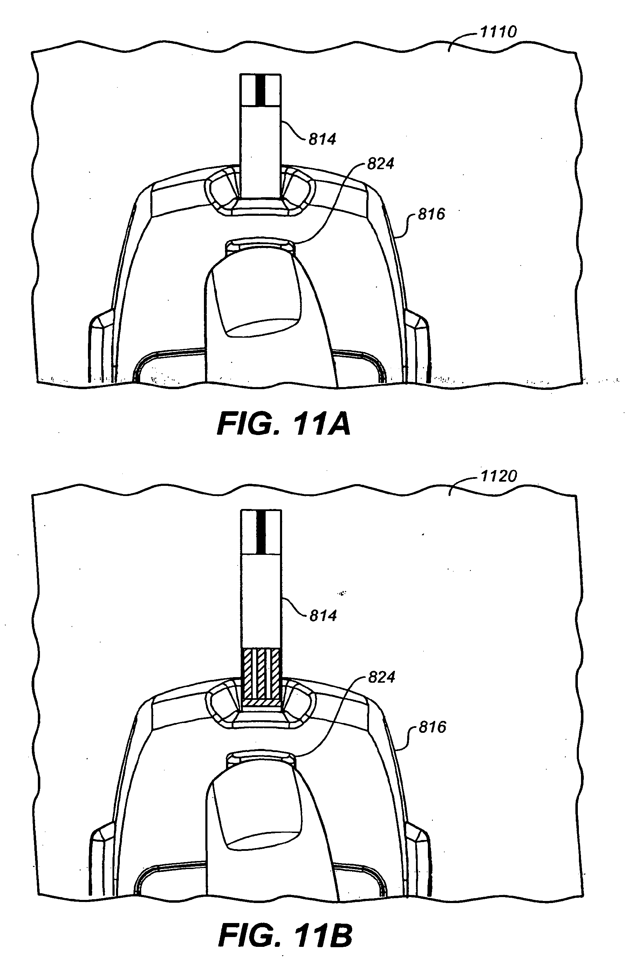

FIGs. l0A through l0E are simplified depictions of portions of tutorial images of a yet further chapter for tutoring a user in the discarding of a used lancet that is referenced in configuration 400 at step 418. FIGs. 11A through 11C are simplified depictions of portions of tutorial images of yet an additional chapter for tutoring a user in the discarding of a used test strip that is referenced in configuration 400 at step 420.

As previously noted, the user interface, microprocessor unit and memory unit of display-based tutorials employed in embodiments of the present invention are operatively linked and configured for event-driven chapter-based display of the tutorial images to a user on visual display 112. An exemplary, but non-limiting, event-driven chapter-based display of tutorial images for which such a display-based tutorial can be configured is depicted in configuration 400 of FIG. 4.

Referring to FIG. 4 and to FIGs. 5A through 11C, an analytical meter according to embodiments of the present invention that is an "off" state (i.e., deactivated, see step 402 of configuration 400) can be activated by a user via various means, such as depression of a tutorial button (e.g., a user-operable tutorial button as described above with respect to FIGs. 2 and 3), automatically via the insertion of an analytical test strip (referred to simply as a "Strip" in FIG. 4) or via any other suitable activation means.

Upon activation of the analytical meter, a microprocessor unit of the display-driven tutorial module determines the unique event that has occurred and then, in cooperation of the memory unit and visual display, displays a chapter of a tutorial that is associated with the determined unique event. For example, if activation has been achieved by depression of a tutorial button, a predetermined opening message is displayed on the visual display (see step 404 of configuration 400). Such an opening message can include, for example, a brand icon, a current date, a current time and a last glucose concentration which was measured.

Moreover, in the configuration of FIG. 4, a second depression of the tutorial button results in the event-driven chapter-based display of tutorial images related to the preparation of a lancet (see step 406 of configuration 400). Such tutorial images are exemplified by FIGs. 5A through 5G, of which:
FIG. 5A is a portion of tutorial image 510 depicting a step in preparing a lancing device 512 that involves removing a lancing device cap 514 with a counter clockwise motion (as depicted by the arrow of FIG. 5A);
FIG. 5B is a portion of a tutorial image 520 depicting a step of preparing lancing device 512 by mounting (in the direction of the arrow of FIG. 5B) a lancet 516 into lancing device 512 with the lancing device cap 514 removed;

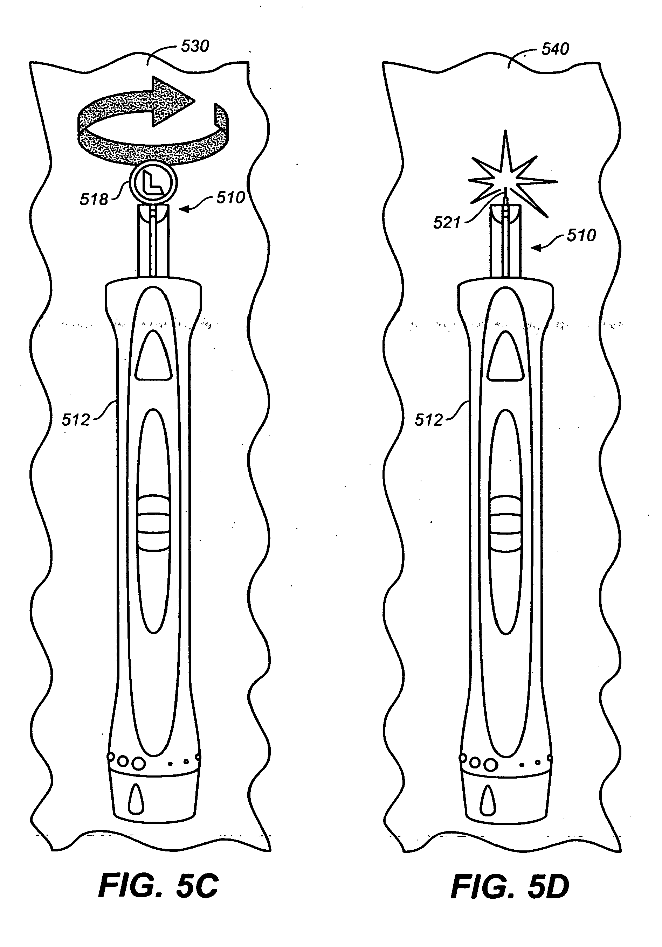

FIG. 5C is a portion of a tutorial image 530 depicting a step of preparing the lancing device by removing a lancet cap 518 using a twisting motion (as depicted by the arrow of FIG. 5C);
FIG. 5D is a portion of a tutorial image 540 depicting a step of preparing the lancing device that highlights (using a starburst image) a sharp lancet tip 521 that was exposed by removal of the lancet cap;

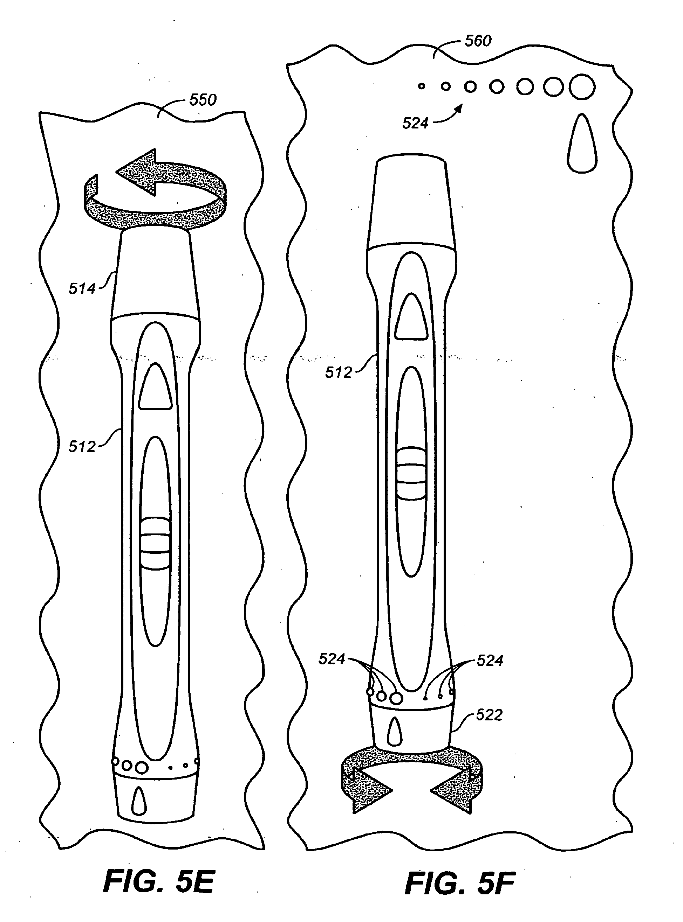

FIG. 5E is a portion of a tutorial image 550 depicting a step of preparing the lancing device by re-attaching the cap using a clockwise motion (as depicted by the arrow of FIG. 5E);
FIG. 5F is a portion of a tutorial image 560 depicting a step of preparing the lancing device by adjusting a depth control mechanism 522 of the lancing device through either a clockwise or counterclockwise motion, with various depth control markings 524 being independently depicted in the upper right-hand corner of tutorial image 560; and

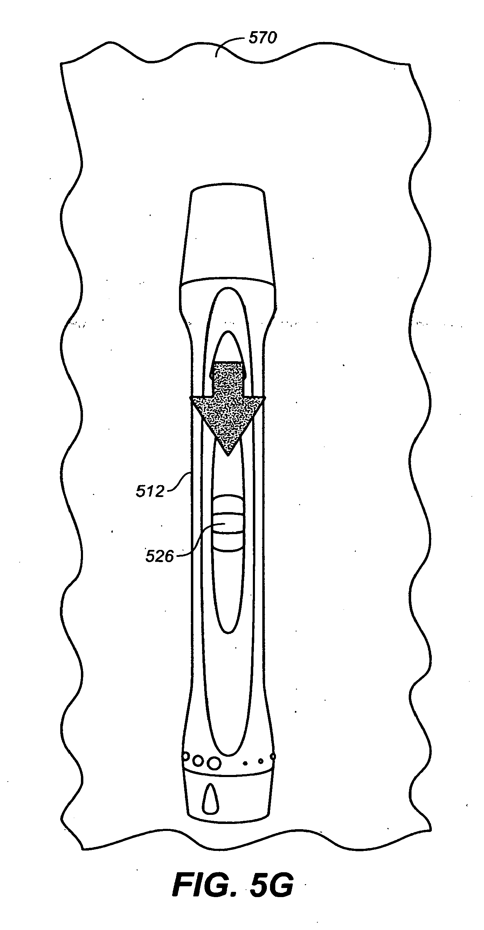

FIG. 5G is a portion of a tutorial image 570 depicting a step of preparing the lancing device by a user cocking (i.e., arming) a launch mechanism 526 (as illustrated by the arrow of FIG. 5G).

Alternatively, if activation has been achieved by insertion of an analytical test strip, the event-driven chapter-based display of tutorial images related to the dosing of blood, i.e., application of a whole blood sample to an analytical test strip, (see step 412 of configuration 400), is displayed to the user. Such tutorial images are exemplified by FIGS. 8A through 8C, of which:
FIG. 8A is a portion of a tutorial image 810 depicting a step of dosing a blood sample by showing a fingertip FT having a drop of blood DB nearby a sample inlet 812 of an analytical test strip 814 inserted in an analytical meter 816;

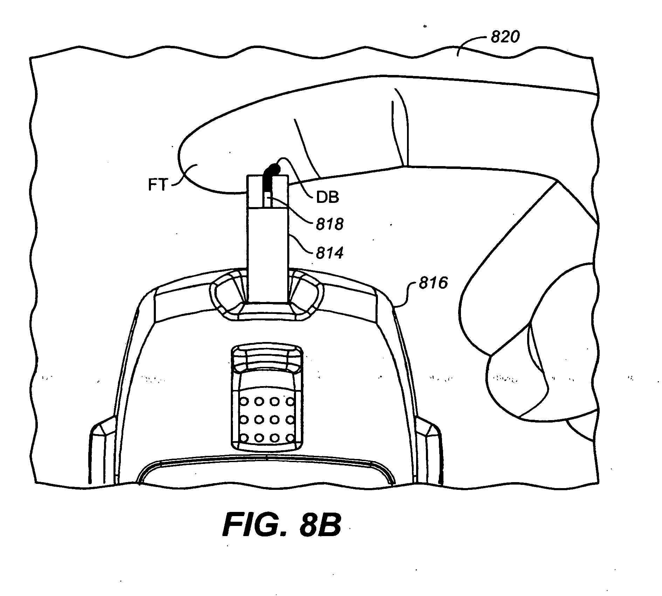

FIG. 8B is a portion of a tutorial image 820 depicting a step of dosing a blood sample by showing a drop of blood DB touching sample inlet 812 such that a sample receiving chamber 818 of analytical test strip 814 is partially filled with blood; and
FIG. 8C is a portion of a tutorial image 830 depicting a step of shows dosing a blood sample by showing sample receiving chamber 818 fully filled with the blood sample BD.

As depicted in FIG. 4, the event-driven chapter-based display of tutorial images related to the dosing of blood, i.e., application of a whole blood sample to an analytical test strip (see step 412 of configuration 400), can also be instigated by the insertion of an analytical test strip into the analytical meter following any of steps 404, 406, 408 and 410 of configuration 400. Moreover, the sequence of images depicted by FIGs. 8A, 8B and 8C can, if desired, be displayed in the manner of an animated sequence that depicts the blood sample BD-being wicked into the analytical test strip 814.

One skilled in the art will recognize that the tutorial images of FIGs. 5A through 11C are exemplary in nature and that other suitable tutorial images can be employed in embodiments of the present invention. For example, the tutorial image within the dashed box of FIG. 8A could itself serve as a tutorial image illustrating a step for dosing a blood sample.

Another event-driven chapter-based display of tutorial images employs a chapter of tutorial images (such as FIGs. 6A-6F) related to strip insertion into an analytical meter (see step 408 of FIG. 4). In the embodiment of FIG. 4 such a display is driven by the unique event of the tutorial button being depressed following step 406. Such tutorial images are exemplified by FIGs. 6A through 6F, of which:
FIG. 6A is a portion of a tutorial image 610 depicting a step of inserting an analytical test strip 814 into an strip port connector 821 of an analytical meter 816 wherein the analytical test strip is outside of strip port connector 821 of analytical meter 816;

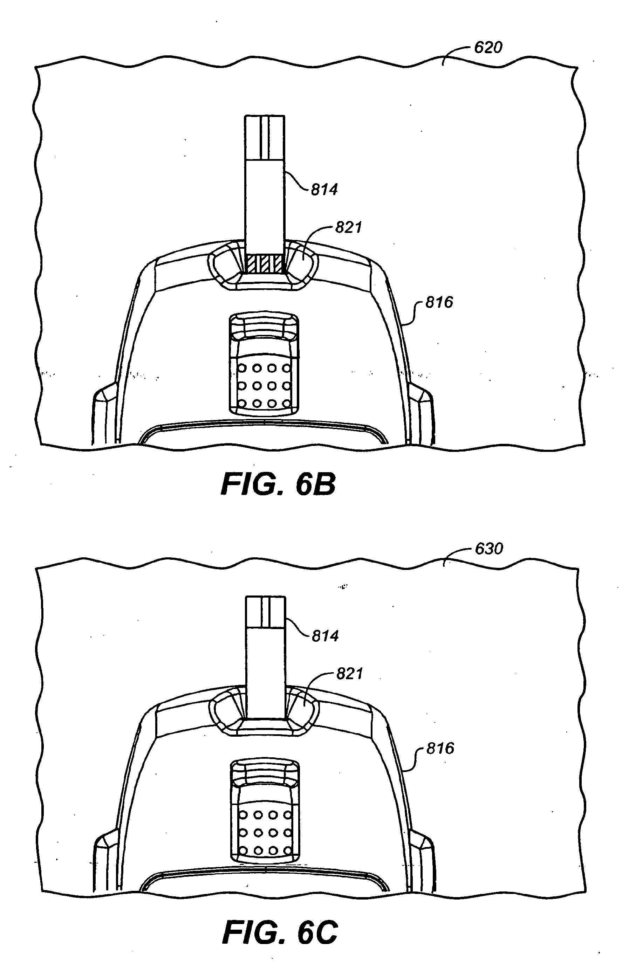

FIG. 6B is a portion of a tutorial image 820 depicting a step of inserting analytical test strip 814 wherein analytical test strip 814 is partially inserted into strip port connector 820;
FIG. 6C is a portion of a tutorial image 830 depicting a step of inserting an analytical test strip 814 wherein analytical test strip 814 is fully inserted into strip port connector 821;

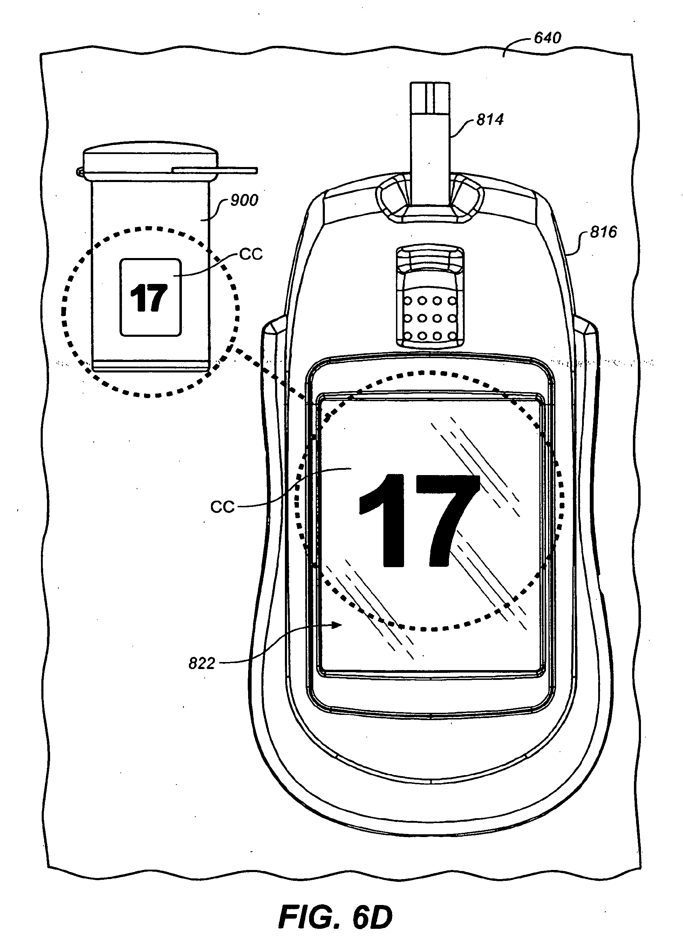

FIG. 6D is a portion of a tutorial image 840 depicting a step of inserting an analytical test strip 814 into an analytical meter 816 that prompts a user to verify that a proper test strip calibration code CC (i.e., the number "17" within the dashed circles of FIG. 6D) has been input to analytical meter 816 by displaying the calibration code on visual display 822 of analytical meter 816 and on an associated vial 900 of analytical test strips;

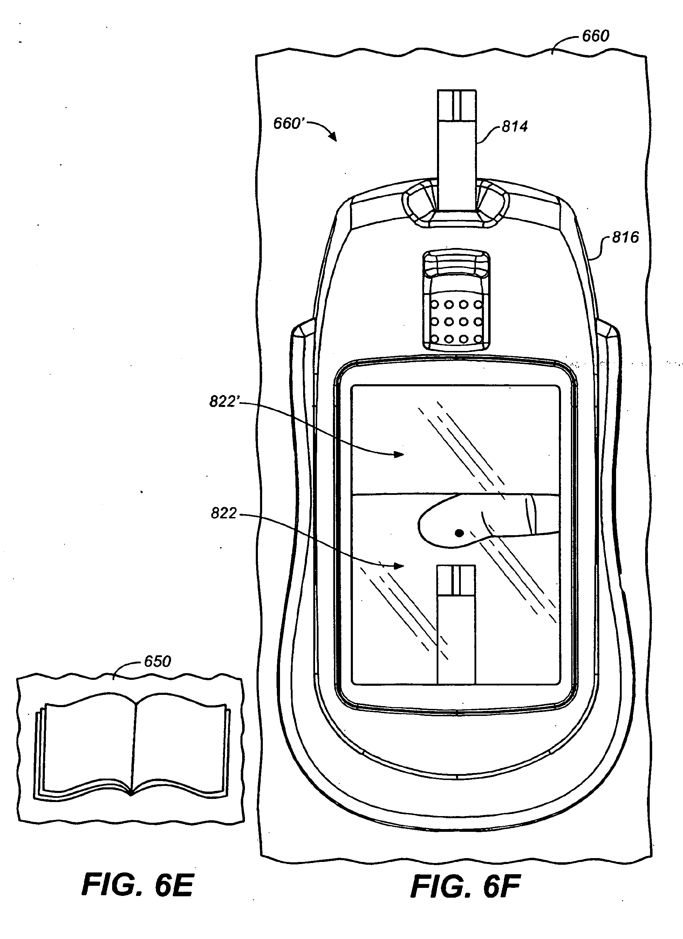

FIG. 6E is a portion of a tutorial image 650 that prompts a user to refer to an operations manual during the insertion of an analytical test strip should such a need arise; and
FIG. 6F is a portion of a tutorial image 660 that prompts a user to verify proper analytical test strip insertion by showing an analytical meter that has an appropriate tutorial image on its visual display (i.e., the image from within the dashed box of FIG. 8A).

If desired, FIGs. 6A through 6F can be displayed on a visual display as a sequence of animated images depicting the insertion of analytical test strip 814 into strip port connector 821. Moreover, image associated text can be displayed along with any the tutorial images. For example, text could be displayed in the upper area 660' of tutorial image 660 and/or on the upper portion 822' of visual display 822 of analytical meter 816.

A further event-driven chapter-based display of tutorial images employs a chapter of tutorial images (such as FIGs. 7A and 7B) related to the lancing of a finger to obtain a whole blood sample (see step 410 of FIG. 4). In the embodiment of FIG. 4 such a display is driven by the unique event of the tutorial button being depressed following step 408. Such tutorial images are exemplified by FIGs. 7A and 7B, of which:
FIG. 7A is a portion of a tutorial image 710 depicting a step of lancing a fingertip FT by placing fingertip FT on top of lancing device 512; and
FIG. 7B is a portion of a tutorial image 720 depicting a step of lancing a fingertip FT that illustrates actuation of launch mechanism 526 by a user's thumb UT.

An additional event-driven chapter-based display of tutorial images employs a chapter of tutorial images (such as FIGs. 9A and 9B) depicts a simulated display of a test by the analytical meter (see step 414 of configuration 400). In the embodiment of FIG. 4 such a display is driven by the unique event of the tutorial button being depressed following step 412 (note, however, that if an actual whole blood sample is dosed following step 412, steps 428 (the display of an actual countdown), 426 (the display of an actual glucose concentration) and, optionally, 402' (deactivation of the analytical meter) will occur). Such tutorial images are exemplified by FIGs. 9A and 9B, of which:
FIG. 9A is a portion of a tutorial image 910 depicting shows a test time count down display 912 wherein the test time has five seconds remaining; and
FIG. 9B is a portion of a tutorial image 920 depicting the display of a glucose concentration as determined by analytical meter.

The chapter of images exemplified by FIGs. 9A and 9B can be of use to a user who desires to review the manner in which a test time count down and glucose concentration are displayed without actually dosing a whole blood sample onto an analytical test strip.

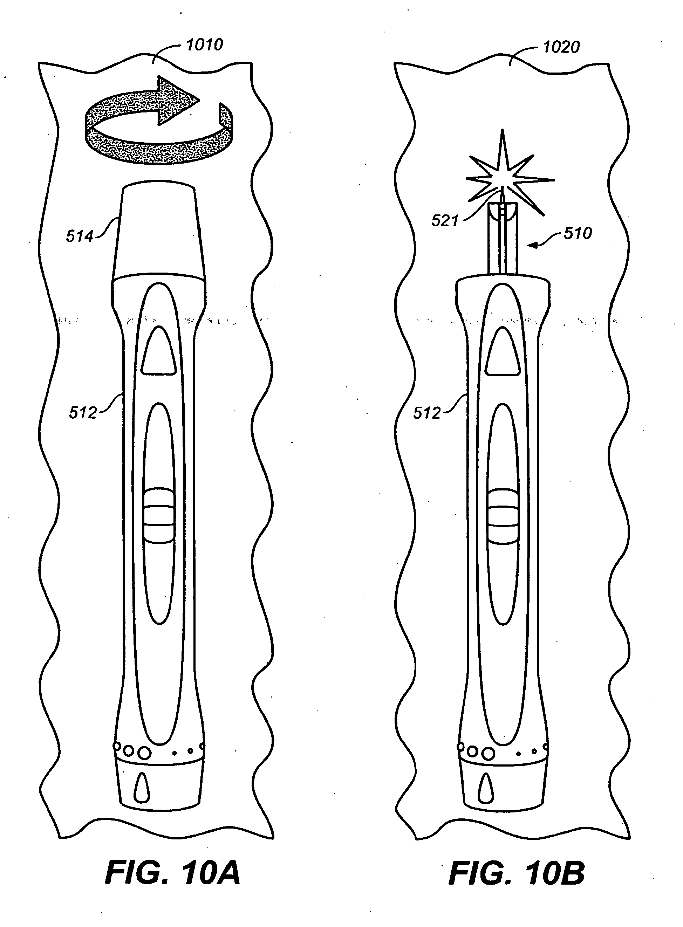

Still a further event-driven chapter-based display of tutorial images employs a chapter of tutorial images (such as FIGs. l0A through l0E) that depict the discarding is a used lancet (see step 418 of configuration 400). In the embodiment of FIG. 4 such a display is driven by the unique event of the tutorial button being depressed following step 414 or the unique event of the tutorial button being depressed following step 426 (note, however, that if a whole blood sample is dosed following step 414, steps 428 (the display of an actual countdown), 426 (the display of an actual glucose concentration) and, optionally, 402' (deactivation of the analytical meter) will occur. Such tutorial images are exemplified by FIGs. 10A through 10E, of which:
FIG. 10A is a portion of a tutorial image 1010 depicting a step of discarding of a used lancet by first removing lancing device cap 514 through rotating it counter clockwise (as depicted by the arrow of FIG. l0A);
FIG. 10B is a portion of a tutorial image 1020 depicting a step of discarding of a used lancet wherein lancing device cap 514 has been removed which has exposed a sharp tip 521;

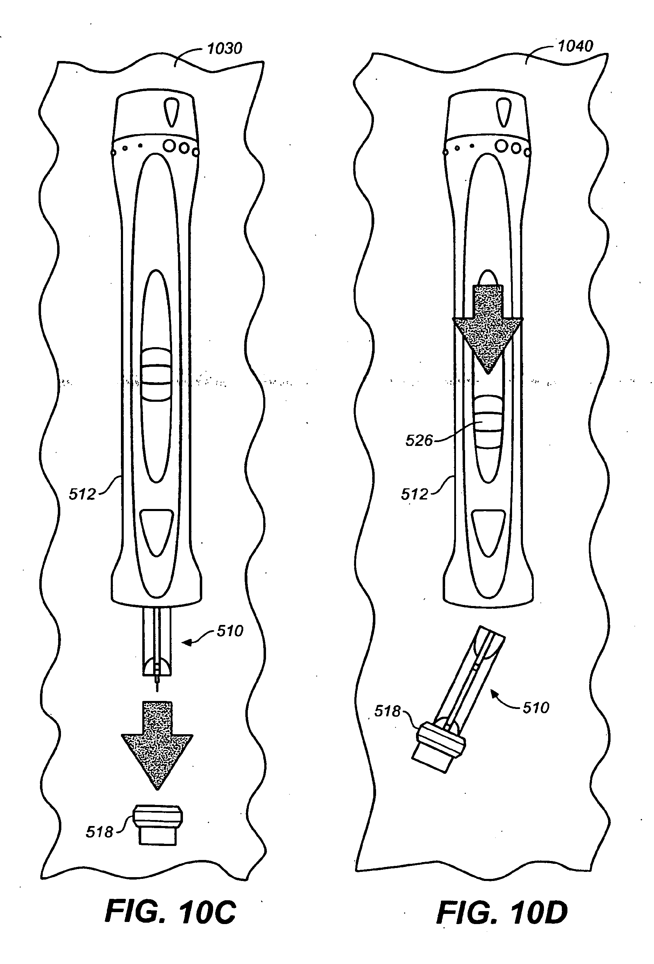

FIG. 10C is a portion of a tutorial image 1030 depicting a step of discarding of a used lancet depicting the attachment of a lancet cap 518 to the sharp tip 521;
FIG. 10D is a portion of a tutorial image 1040 depicting a step of discarding of a used lancet by ejecting the used lancet from lancing device 512; and

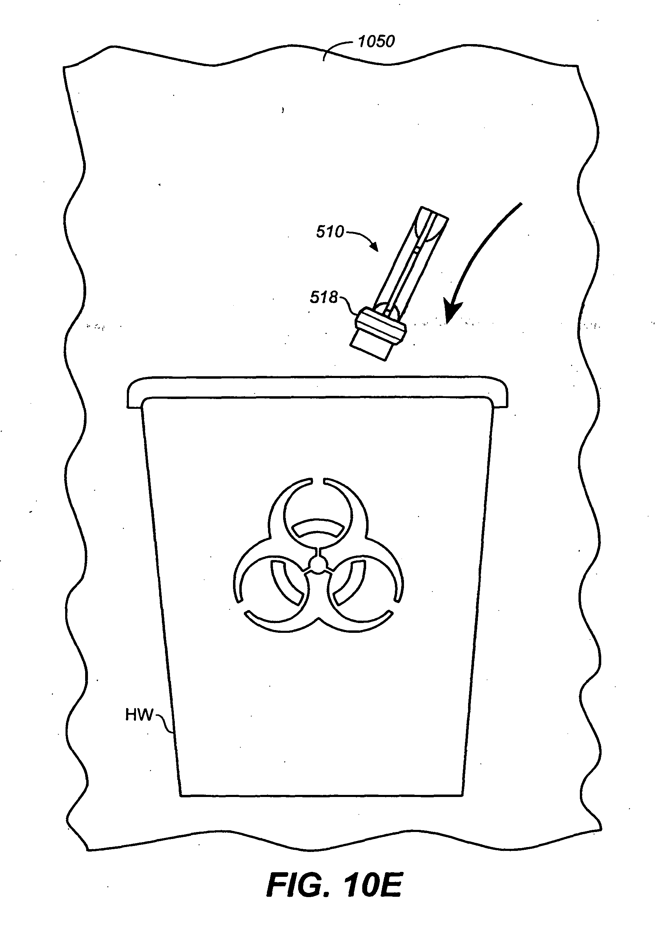

FIG. 10E is a portion of a tutorial image 1050 depicting a step of discarding of a used lancet by illustrating the used lancet being ejected into a hazardous waste container HW.

Still an additional event-driven chapter-based display of tutorial images employs a chapter of tutorial images (such as FIGs. 11A through 11C) that depict the discarding of a used analytical test strip (see step 420 of configuration 400). In the embodiment of FIG. 4 such a display is driven by the event of the tutorial button being depressed following step 418 (note, however, that if a whole blood sample is dosed following step 418, steps 428 (the display of an actual countdown), 426 (the display of an actual glucose concentration) and, optionally, 402' (deactivation of the analytical meter) will occur. Such tutorial images are exemplified by FIGs. 11A through 11C of which:
FIG. 11 A is a portion of a tutorial image 1110 of a step for discarding a used test strip by starting to push an ejector button 824 of analytical meter 816;
FIG. 11B is a portion of a tutorial image 1120 of a step for discarding a used test strip by fully pushing ejector button 824; and

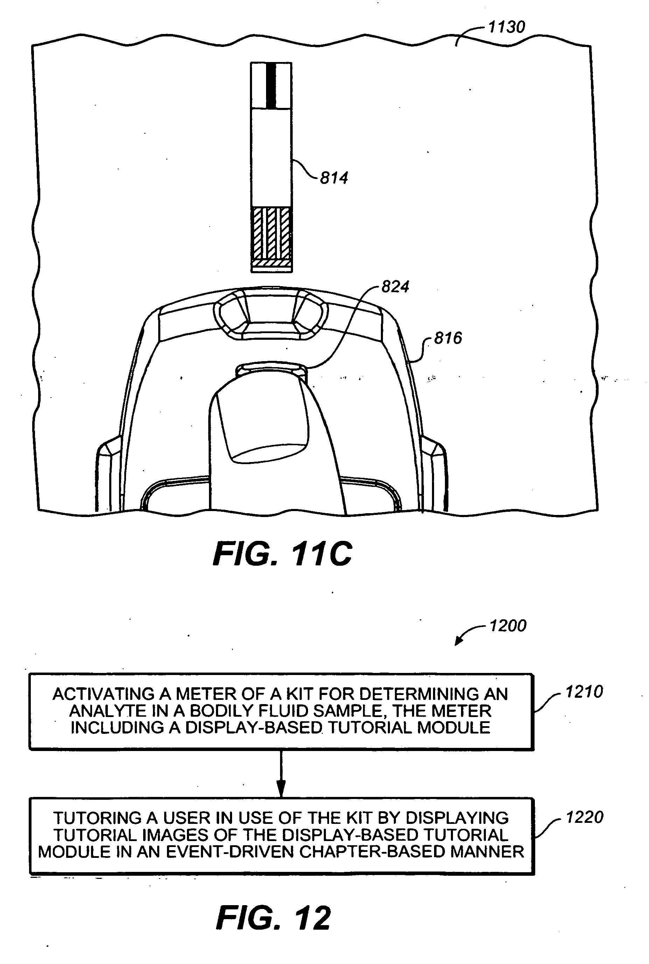

FIG. 11C is a portion of a tutorial image 1130 of a step for discarding a used test strip illustrating a fully ejected used analytical test strip.

If a whole blood sample is dosed following step 420, steps 428 (the display of an actual countdown), 426 (the display of an actual glucose concentration) and, optionally, 402' (deactivation of the analytical meter) will occur. After step 420, depression of the tutorial button will result in the analytical meter determining whether an actual or simulated test was performed (see step 422 of configuration 400). If the test meter result was a simulated test, then an end message will be displayed (see step 424 of configuration 400). If the test result was an actual test result, then steps 426 and 402' of configuration 400 will occur.

As described above, each chapter of tutorials according to the present invention is assigned to one or more unique events that can occur during use of a kit or analytical meter for the determination of an analyte in a bodily fluid sample. The unique events can be, for example, insertion of an analytical test strip into an analytical meter following other predetermined steps or the dosing (i.e., application) of a bodily fluid sample onto the analytical test strip following other predetermined steps. Moreover, should a user be uncertain about how to proceed during the course of conducting an analyte determination, the user can initiate an appropriate event-driven chapter-based display of tutorial images by depressing a user-operable tutorial button. Since the tutorial images are event-driven and chapter-based, they are relevant to the issues a user may be encountering following each of the unique events that occur in the course of performing a determination.

Once apprised of the present disclosure, one skilled in the art will recognize that events other than those described herein can be used as a basis for the event-driven chapter-based display of tutorial images. For example, events can be based on an analytical meter recognizing that i) sample receiving chamber is only partially filled with blood, ii) ambient temperature is greater than about 45 degrees Celsius, iii) ambient temperature is less than about 4 degrees Celsius, and iv) a battery of the analytical meter is about to be completely discharged. For each of these events, tutorial images can be devised and assigned to a chapter of a tutorial.

FIG. 12 is a flow diagram depicting stages in a method 1200 for tutoring a user in use of a kit for determining an analyte (such as glucose) in a bodily fluid sample (e.g., a whole blood sample) according to an exemplary embodiment of the present invention. Method 1200 includes activating a meter of a kit for determining an analyte in a bodily fluid sample, as set forth in step 1210.

The meter that is activated in step 1210 includes a display-based tutorial module with a user interface (that has a visual display), a memory unit and a microprocessor unit. A tutorial that has a plurality of chapters is stored within the memory unit. Moreover, each of the plurality of chapters contains at least one tutorial image depicting use of the kit. The microprocessor unit is configured for controlling and coordinating at least the user interface and the memory unit. Moreover, the user interface, microprocessor unit and memory unit are operatively linked and configured for event-driven chapter-based display of the tutorial images to a user on the visual display.

At step 1220 of method 1200, the user is tutored in use of the kit by displaying the tutorial images in an event-driven chapter-based manner on the visual display of the user interface.

Once apprised of the present disclosure, one skilled in the art will recognize that methods according to embodiments of the present invention can include steps that carry out functional characteristics of kits and analytical meters according to embodiments the present invention as described herein. For example, the activating step of methods according to the present invention can include the activation of any suitable meter described with respect to embodiments of the present invention.

Analytical meters, kits and methods according to the present invention are beneficial in that they provide a user with a tutorial in an easy-to-use and visual format (i.e., an event-driven chapter-based configuration). The event-driven feature eliminates the potential for tedious navigation through an entire tutorial or user operating manual when only a particular portion of the tutorial (i.e., a chapter) is relevant to a user. The event-driven and chapter-based features of analytical meters, kits and methods according to the present invention, therefore, provide a rational means for displaying only relevant tutorial images to a user.

It should be understood that various alternatives to the embodiments of the invention described herein may be employed in practicing the invention. It is intended that the following claims define the scope of the invention and that structures and methods within the scope of these claims and their equivalents be covered thereby.

## Claims

1. A method for tutoring a user in use of a kit for determining an analyte in a bodily fluid sample, the method comprising:
activating an analytical meter of a kit for determining an analyte in a bodily fluid sample, the analytical meter including:
a display-based tutorial module with:
a user interface that includes a visual display;
a memory unit storing a tutorial, the tutorial having a plurality of chapters with each of the plurality of chapters containing at least one tutorial image depicting use of the kit; and
a microprocessor unit configured for controlling and coordinating at least the user interface and the memory unit,
wherein the user interface, microprocessor unit and memory unit are operatively linked and configured for event-driven chapter-based display of the tutorial images to a user on the visual display, and
tutoring the user on use of the kit by displaying the tutorial images in an event-driven chapter-based manner.

2. The method of claim 1 wherein the analytical meter is configured for determination of glucose in a whole blood sample.

3. The method of claim 1 wherein display-based tutorial module includes a user-operable tutorial button, and
wherein the event-driven chapter-based display of the tutorial images in the tutoring step includes at least one event-driven chapter-based display driven by an event of the user depressing the user operable tutorial button.

4. The method of claim 1 wherein the event-driven chapter-based display of the tutorial images in the tutoring step includes a sequence of animated tutorial images.

5. The method of claim 1 wherein the visual display is configured to display an analyte concentration.

6. The method of claim 1 wherein the event-driven chapter-based display of tutorial images in the tutoring step is based on an analytical strip insertion into the analytical meter event.

7. The method of claim 1 wherein the event-driven chapter-based display of tutorial images in the tutoring step is based on an bodily fluid dosing event.

8. The method of claim 1 wherein the event-driven chapter-based display of the tutoring step includes display of tutorial images depicting bodily fluid dosing.

9. The method of claim 1 wherein the event-driven chapter-based display of the tutoring step includes display of tutorial images depicting analytical test strip insertion into the analytical meter.

10. The method of claim 1 wherein the event-driven chapter-based display of the tutoring step includes display of tutorial images depicting a simulated determination of an analyte in a bodily fluid sample by the analytical meter.

## Patentansprüche

1. Verfahren zur Anleitung eines Benutzers in der Verwendung eines Kits zur Bestimmung eines Analyts in einer Körperflüssigkeitsprobe, umfassend:
Aktivieren eines Analysegeräts eines Kits zur Bestimmung eines Analyts in einer Körperflüssigkeitsprobe, wobei das Analysegerät enthält:
ein anzeigebasiertes Anleitungsmodul mit:
einer Benutzerschnittstelle, die eine optische Anzeige enthält;
einer Speichereinheit, die eine Anleitung speichert, wobei die Anleitung eine Vielzahl von Kapiteln aufweist, wobei jedes der Vielzahl von Kapiteln mindestens ein Anleitungsbild enthält, das die Verwendung des Kits darstellt; und
einer Mikroprozessoreinheit, die zur Steuerung und Koordinierung von zumindest der Benutzerschnittstelle und der Speichereinheit konfiguriert ist,
wobei die Benutzerschnittstelle, Mikroprozessoreinheit und Speichereinheit operativ verbunden und für ereignisgesteuerte, kapitelbasierte Anzeige der Anleitungsbilder für einen Benutzer auf der optischen Anzeige konfiguriert sind,
Unterweisen des Benutzers hinsichtlich der Verwendung des Kits durch Anzeigen der Anleitungsbilder in einer ereignisgesteuerten, kapitelbasierten Weise.

2. Verfahren nach Anspruch 1, **dadurch gekennzeichnet, dass** das Analysegerät zur Bestimmung von Glukose in einer Vollblutprobe konfiguriert ist.

3. Verfahren nach Anspruch 1, **dadurch gekennzeichnet, dass** das anzeigebasierte Anleitungsmodul einen von einem Benutzer betätigbaren Anleitungsknopf enthält und dass die ereignisgesteuerte, kapitelbasierte Anzeige der Anleitungsbilder in dem Anleitungsschritt mindestens eine ereignisgesteuerte, kapitelbasierte Anzeige enthält, die durch Drücken des von einem Benutzer betätigbaren Anleitungsknopfes gesteuert wird.

4. Verfahren nach Anspruch 1, **dadurch gekennzeichnet, dass** die ereignisgesteuerte, kapitelbasierte Anzeige der Anleitungsbilder in dem Anleitungsschritt eine Abfolge von animierten Anleitungsbildern enthält.

5. Verfahren nach Anspruch 1, **dadurch gekennzeichnet, dass** die optische Anzeige zum Anzeigen einer Analytkonzentration konfiguriert ist.

6. Verfahren nach Anspruch 1, **dadurch gekennzeichnet, dass** die ereignisgesteuerte, kapitelbasierte Anzeige von Anleitungsbildern in dem Anleitungsschritt auf einer Analysestreifeneinführung in das Analysegerät basiert.

7. Verfahren nach Anspruch 1, **dadurch gekennzeichnet, dass** die ereignisgesteuerte, kapitelbasierte Anzeige von Anleitungsbildern in dem Anleitungsschritt auf einem Körperflüssigkeitsdosierereignis basiert.

8. Verfahren nach Anspruch 1, **dadurch gekennzeichnet, dass** die ereignisgesteuerte, kapitelbasierte Anzeige des Anleitungsschrittes Anzeige von Anleitungsbildern enthält, die Körperflüssigkeitsdosierung darstellen.

9. Verfahren nach Anspruch 1, **dadurch gekennzeichnet, dass** die ereignisgesteuerte, kapitelbasierte Anzeige des Anleitungsschrittes Anzeige von Anleitungsbildern enthält, die Analyseteststreifeneinführung in das Analysegerät darstellen.

10. Verfahren nach Anspruch 1, **dadurch gekennzeichnet, dass** die ereignisgesteuerte, kapitelbasierte Anzeige des Anleitungsschrittes Anzeige von Anleitungsbildern enthält, die eine simulierte Bestimmung eines Analyts in einer Körperflüssigkeitsprobe durch das Analysegerät darstellen.

## Revendications

1. Procédé pour former un utilisateur à l'utilisation d'un kit servant à déterminer une substance à analyser dans un échantillon de fluide corporel, le procédé comprenant les étapes consistant à :
> activer un dispositif de mesure analytique d'un kit servant à déterminer une substance à analyser dans un échantillon de fluide corporel, le dispositif de mesure analytique comprenant :
> un module didactique basé sur l'affichage comportant :
> une interface utilisateur comprenant un écran d'affichage visuel ;
> une unité de mémoire stockant un didacticiel, le didacticiel comportant une pluralité de chapitres, chaque chapitre de la pluralité de chapitres contenant au moins une image didactique décrivant l'utilisation du kit ; et
> un microprocesseur configuré pour commander et coordonner au moins l'interface utilisateur et l'unité de mémoire,
dans lequel l'interface utilisateur, le microprocesseur et l'unité de mémoire sont fonctionnellement reliés et configurées pour l'affichage événementiel par chapitre des images didactiques à l'attention d'un utilisateur sur l'écran d'affichage visuel, et
> former l'utilisateur à l'utilisation du kit en affichant les images didactiques d'une façon événementielle par chapitre.

2. Procédé selon la revendication 1, dans lequel le dispositif de mesure analytique est configuré pour déterminer le glucose dans un échantillon de sang total.

3. Procédé selon la revendication 1, dans lequel le module didactique basé sur l'affichage comprend un bouton didactique pouvant être actionné par l'utilisateur, et dans lequel l'affichage événementiel par chapitre des images didactiques à l'étape de formation comprend au moins un affichage événementiel par chapitre commandé par un événement de l'utilisateur appuyant sur le bouton didactique pouvant être actionné par l'utilisateur.

4. Procédé selon la revendication 1, dans lequel l'affichage événementiel par chapitre des images didactiques à l'étape de formation comprend une séquence d'images didactiques animées.

5. Procédé selon la revendication 1, dans lequel l'écran d'affichage visuel est configuré pour afficher une concentration de substance à analyser.

6. Procédé selon la revendication 1, dans lequel l'affichage événementiel par chapitre d'images didactiques à l'étape de formation est basé sur un événement d'insertion de bande analytique dans le dispositif de mesure analytique.

7. Procédé selon la revendication 1, dans lequel l'affichage événementiel par chapitre d'images didactiques à l'étape de formation est basé sur un événement de dosage de fluide corporel.

8. Procédé selon la revendication 1, dans lequel l'affichage événementiel par chapitre à l'étape de formation comprend l'affichage d'images didactiques décrivant le dosage de fluide corporel.

9. Procédé selon la revendication 1, dans lequel l'affichage événementiel par chapitre à l'étape de formation comprend l'affichage d'images didactiques décrivant l'insertion d'une bande test analytique dans le dispositif de mesure analytique.

10. Procédé selon la revendication 1, dans lequel l'affichage événementiel par chapitre à l'étape de formation comprend l'affichage d'images didactiques décrivant une détermination simulée d'une substance à analyser dans un échantillon de fluide corporel par le dispositif de mesure analytique.
